# EUROPEAN PATENT SPECIFICATION

(11) **EP 0 421 780 B1**
(45) Date of publication and mention of the grant of the patent: **02.01.1997**
(21) Application number: 90310869.4
(22) Date of filing: 04.10.1990
(51) Int. Cl.: A61F 2/72

(54) **Multifunction control of a prosthetic limb using syntactic analysis of the dynamic myoelectric signal patterns associated with the onset of muscle contraction**
Multifunktionssteuerung eines Kunstgliedes durch syntaktische Analyse dynamischer myoelektrischer Signalmuster in Verbindung mit dem Auftreten der Muskelkontraktion
Commande multifonctions pour membre artificiel par analyse syntactique de modèles de signaux dynamiques myoélectriques associés avec le début de contraction musculaire

(30) Priority: 04.10.1989 GB 8922368
(43) Date of publication of application: 10.04.1991
(73) Proprietor: Hugh Steeper Limited, London SW15 4LB (GB)
(72) Inventor: Hudgins, Bernard Sanford, Castle Acres, Fredericton NB, E3B 5WS (CA)
(74) Representative: Heath, Derek James

(56) References cited:
- FR-B- 2 325 098
- US-A- 4 030 141
- MEDICAL & BIOLOGICAL ENGINEERING & COMPUTING vol. 23, no. 1, January 1985, pages 8-14, Staines, Middlesex, GB; L. PHILIPSON: "Adaptable myoelectric prosthetic control with functional visual feedback using microprocessor techniques"
- IEEE/SEVENTH ANNUAL CONFERENCE OF THE ENGINEERING IN MEDICINE AND BIOLOGY SOCIETY vol. 2, September 1985, pages 647,648, Chicago, US; J.C. BECKER et al.: "Myoelectric Control of Fingers in a Prosthetic Hand"

## Description

This invention relates to the multifunction control of a prosthetic limb using syntactic analysis of the dynamic myoelectric signal patterns associated with the onset of muscle contraction.

The present invention constitutes a new approach to the control of an artificial limb in which the dynamic nature of the onset of muscle contraction, as reflected in the associated myoelectric signal, is analysed to extract control parameters. This is a complete departure from the present technique which monitors a single variable of the myoelectric signal to determine the state of the control system. For example, in the amplitude coded systems of Otto Bock, University of New Brunswick and several others, the mean absolute value of the myoelectric signal is used to control the on/off state of the prosthetic limb or its speed of operation. Rate sensitive systems have been implemented which determine the system state by the value of the signal after some set integration period. These existing systems look only at the static nature of some measure of the myoelectric signal and not at the dynamic structure in the signal itself. The number of functions which can be controlled by such systems using a single myoelectric channel is limited to three (e.g. hand open, hand close and hand at rest). The new approach presented here overcomes this limitation by selecting limb function based on the dynamic nature of the contraction. True multifunction control is possible due to the great number of distinct patterns which can be generated reliably by varying the intent of the contraction, while pattern classification for function selection can be accomplished by a number of techniques.

In Medical & Biological Engineering & Computing vol 23, no. 1, January 1985, pages 8-14, Staines, Middlesex, GB; L. Philipson: "Adaptable myoelectric prosthetic control with functional visual feedback using microprocessor techniques", there is described a method of controlling a prosthetic limb using myoelectric pulses. Each pulse is generated whenever a myoelectric signal (called MEG) supplied by one electrode exceeds a threshold value. The number of pulses during a fixed time interval permits classification of the MEG signal with each class representing a movement of the prosthesis.

In addition, FR-A-2 325 098 describes a method of controlling a prosthetic limb in which an amplified myoelectric signal is compared to two threshold values in order to activate four different outputs D, E, F and G. Since the threshold values are smaller than the absolute myoelectric signal value, the transient wave forms are monitored to control the artificial limb.

The aim of the present invention is to devise a myoelectric control system wherein use is made of dynamic information in the myoelectric signal for classifying samples of this signal by determining a string of descriptors which are obtained by dividing the sample in segments and calculating primitive descriptors for each segment. Selection of the limb functions in accordance with the pattern class determined is thus made possible. Such a system is not disclosed in any of the prior art discussed above.

The present invention therefore starts from the two prior documents specifically discussed above and is directed to a system having the features set forth in the characterised portion of claim 1.

Preferred features are set forth in the subsidiary claims.

The invention will be explained further, by way of example, with reference to the accompanying drawings, in which :
Figure 1 is a block diagram of one form of myoelectric control system in accordance with the invention;
Figure 2a shows graphs of myoelectric burst patterns for wrist extension;
Figure 2b shows graphs of myoelectric burst patterns for elbow flexion;
Figure 2c shows graphs of myoelectric burst patterns for forearm supination;
Figure 2d shows graphs of myoelectric burst patterns for forearm pronation; and
Figure 2e illustrates examples of each of the four contraction types of Figures 2a to 2d. The neural net work was able to classify patterns of this type using the statistics obtained from 5 segments (dotted vertical lines) with greater than 90% success.

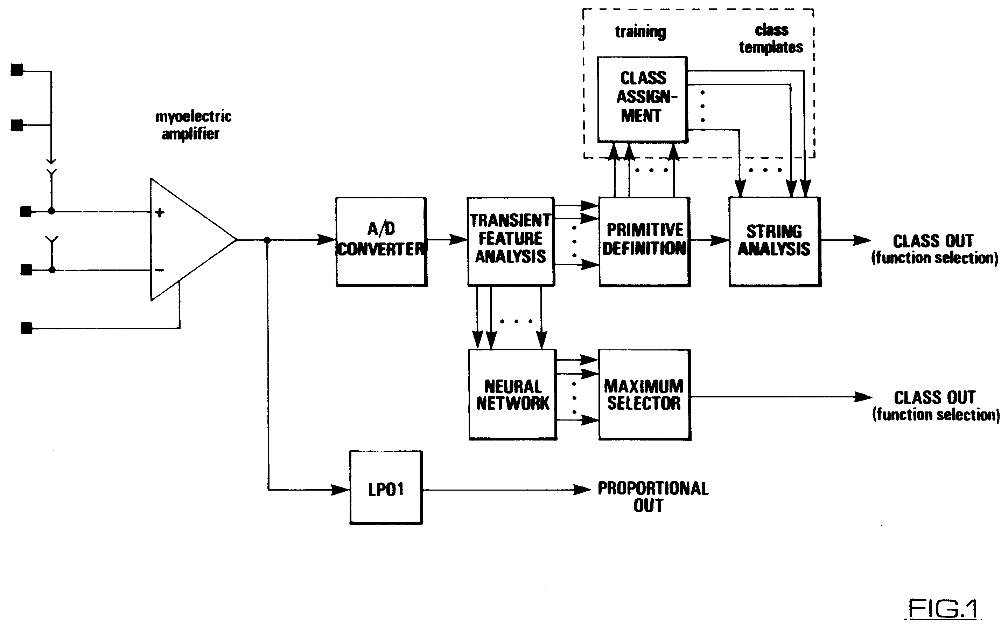

In the new myoelectric control system shown in Figure 1, the myoelectric signal is detected using standard surface electrodes, although the burst patterns can, if desired, be enhanced using split electrodes with multiple active sites. The signal is amplified and band limited using a standard myoelectric amplifier, the myoelectric signal being monitored continuously. The resulting signal is sampled at a rate of 1kHz to provide a discrete representation of the signal. This stream of discrete values is then analysed typically in 40ms (40 sample) segments. Features such as mean absolute value, zero crossings, difference between mean absolute value in successive segments, or other measures, are calculated for each segment. These features are analysed to determine a representative primitive or descriptor for the segment. Several consecutive segments of the signal are analysed similarly to produce a string of descriptors. The sequence of descriptors for each pattern class (i.e. limb function), is determined through a period of system training. An unknown descriptor string can then be analysed to determine which pattern class it resembles most closely. This classification can be done using any number of distance measures as described in the syntactic pattern recognition literature. Because function selection is determined from information at the onset of the contraction, once a function is selected it can be controlled in a proportional manner using a signal from the same myoelectric channel.

Alternatively, the known descriptor sequences can be used to train a three-layer neural network using a back propagation algorithm. The unknown sequences are then classified using this trained network. Inputs to the neural network can include statistics calculated from the total signal as well as the segment statistics. In this way the best features of previous systems can be used to enhance the classification of the new control scheme.

In the particular system shown in Figure 1, features are calculated for five 40ms segments, and a function is activated when the string analysis detects a known string. When a function is selected it is controlled proportionally using the signal from the low pass output (LP01), and the function continues until LP01 falls below a selected threshold. It is to be understood that inputs to neural network are features from several consecutive segments. Either syntactic analysis or the neural network implementation is chosen for function classification.

In essence, therefore, the new approach illustrated in Figure 1 increases the amount and quality of information used to determine limb function from a single myoelectric channel. In other words, the invention aims to increase the number of functions of an artificial limb which can be controlled by a single myoelectric channel. The novel aspect of this invention is not the classification scheme but rather the use of the dynamic information in the signal to enhance classification. This approach can be used to classify other signals with properties similar to the myoelectric signal. These include myoacoustic and seismic waveforms. Although syntactic analysis has been used to some extent on the latter waveform type there is no mention in the literature of mapping the statistical properties onto a neural network.

A myoelectric control system based on this approach must be trained to recognize the individual patterns generated by each user. This requires a training period in which the user repetitively generates patterns of known class. The control system uses the parameters extracted from these patterns to define the descriptors for each distinct pattern class. The training period will have to be repeated whenever the limb is put on. Once the descriptors are determined for the pattern classes, retraining can begin with these values. In this way retraining will be much quicker than the original training period. This training, however, does provide the user with the flexibility of using those control contractions which he or she can generate best, and to alter these to redefine the control patterns.

Preliminary experiments have shown that a pattern classification based on the proposed scheme is feasible. A computer implementation resulted in a correct classification rate of over 95% for a 5-state, four-function control system and over 80% for a 7-state, 6-function system. These results were obtained with very little operator training and it is expected that familiarity with the system will give even better results. The experimental system was based on a neural network implementation of the proposed scheme as outlined in the algorithm given below. A scheme based on syntactic analysis of the myoelectric patterns is being implemented. Work is continuing to determine which parameters of the myoelectric patterns give the best classification.

### ALGORITHM for the NEW MYOELECTRIC CONTROL SYSTEM

1. Begin system training
   -M=1
   (a.)loop 1
   -P=1
   -for contraction type M.
   (b.)loop 2
   -initialize system.
   -set N=1.
   -collect 40 samples of myoelectric data.
   -check sum:if sum<threshold1 drop oldest sample add next; check sum.
   -calculate statistics on those 40 samples, assign to segment N.
   -increment N
   (c.)loop 3
   -collect 40 samples of myoelectric data.
   -calculate statistics; assign to segment N.
   (d.)if N<desired length loop; increment N; goto(c.).
   (e.)else store segment statistics with associated contraction type M.
   (f.)increment P. if P<desired # of training sets goto(b.).
   (g.)else increment contraction type M.
   (h.)If M< desired number of types go to (a.).
   (i.)else train neural network on PxM training sets.
   (j.)store weight array calculated during neural network training.
2. Real Time Operation
   (a.)initialize system.
   -N=1
   (b.)collect 40 samples of myoelectric data.
   (c.)check sum:if sum<threshold1 drop oldest sample add next; check sum.
   (d.)calculate segment N statistics.
   (e.)increment N; if N<desired length goto(b.).
   (f.)use segment statistics as input to trained neural network.
   (g.)choose maximum output of the neural network as function selector.
   (h.)control this function proportionally using level from filtered MES.
   (i.)if level<on threshold goto(a.).

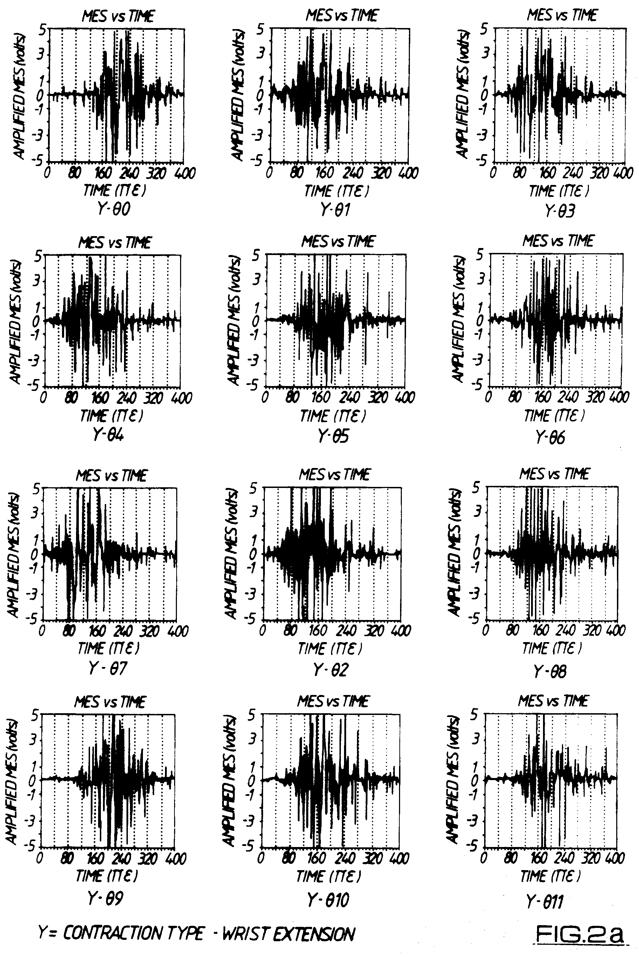

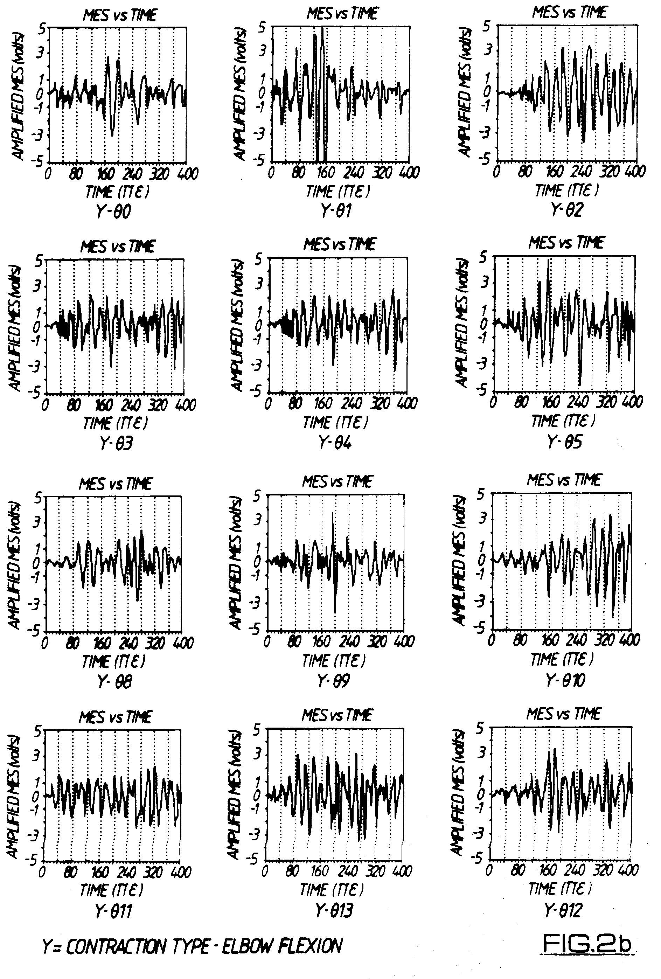

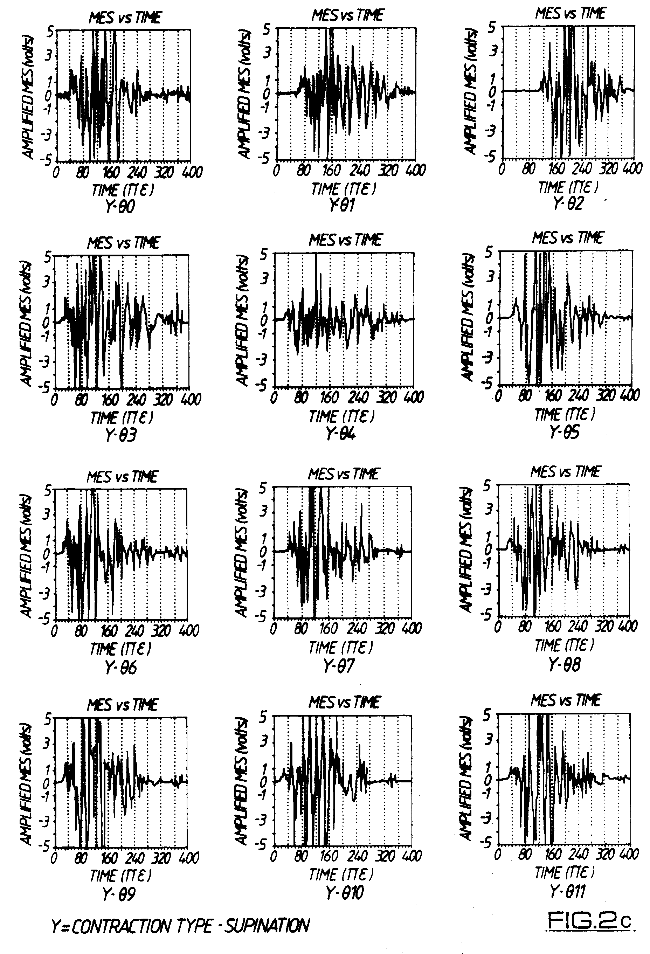

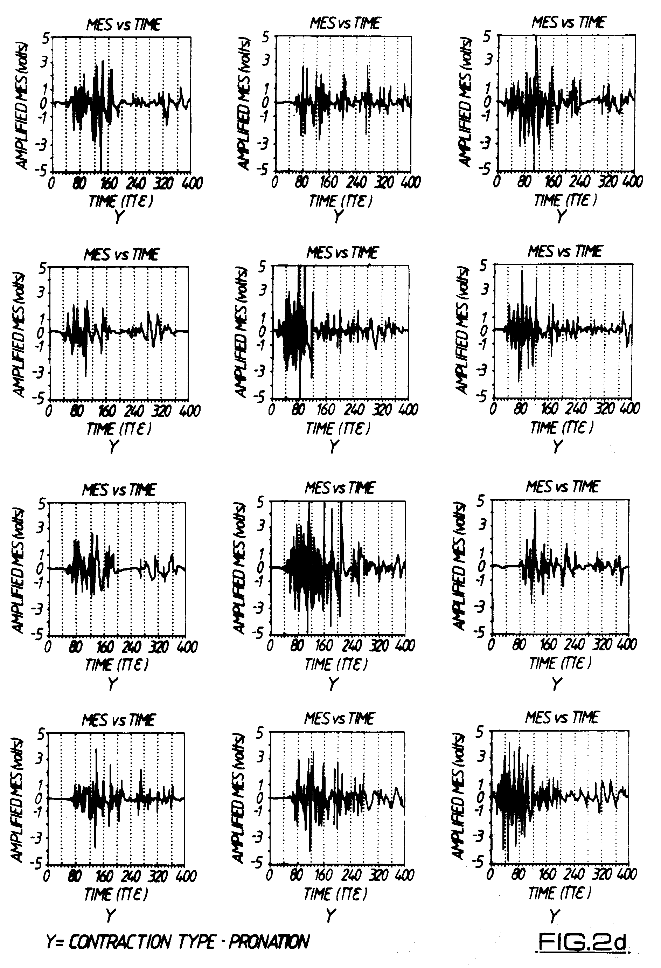

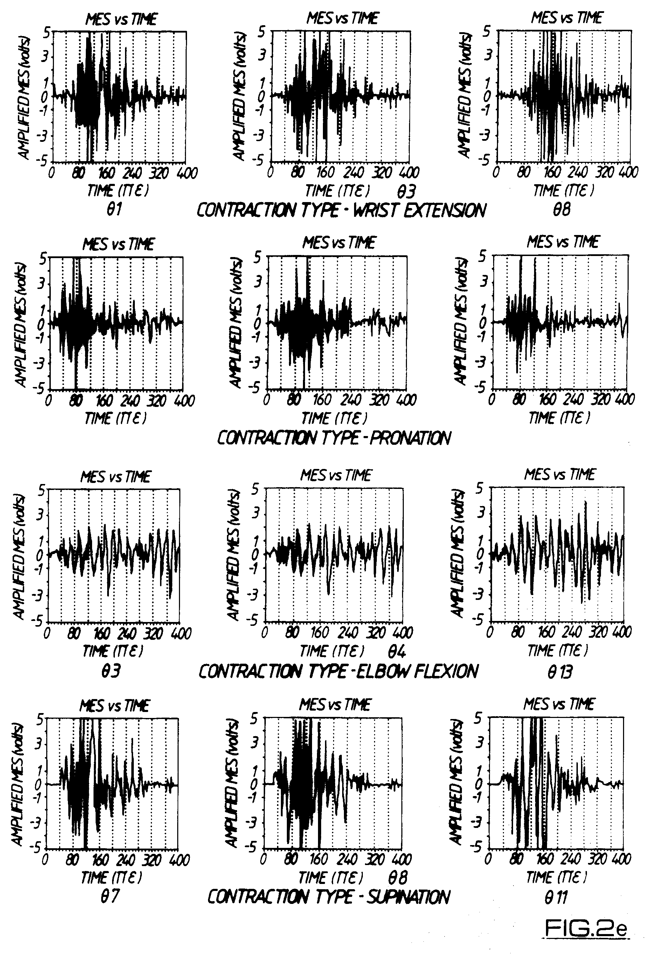

Figures 2a to 2d show the dynamic patterns of myoelectric signal which accompanies the onset of muscle contraction. Each Figure comprises 12 graphs illustrating a series of 12 contractions showing the reproducibility of the inherent structure in the myoelectric burst patterns for each contraction type. Figure 2e is a composition of 3 repeats for each of 4 contraction types. It is the unique characteristics extracted from these patterns for contractions which vary in intent, (e.g. elbow flexion, supination, etc.) which determine pattern class. The experimental system extracted zero crossings and mean absolute value for each 40 ms segment of myoelectric signal, and the difference in the mean absolute value in sucessive segments. The values of these parameters for 5 consecutive segments (200ms) were used as inputs to a three-layer neural network. The network was trained using a back propagation algorithm.

Only subjects with intact musculature have so far been used as operators of this system. It is necessary to demonstrate successful operation with amputee musculature, and results from amputee operators will be forthcoming shortly.

Other researchers have described multifunction control systems based on pattern recognition of the myoelectric signal. These, however, have either been a time series analysis of the steady state signal or a classical discriminant analysis of steady state myoelectric signal features. The present system differs substantially from this in that it uses the dynamic information contained in the signal from the onset of the contraction. As well, this information can be coded in a way which allows either syntactic analysis or a neural network inplementation.

## Claims

1. A myoelectric control system for a prosthetic limb comprising means adapted to operate in dependence on the dynamic information contained in a myoelectric signal (MES) associated with the onset of muscle contraction, the system having converter means (A/D Converter in Fig. 1) for producing digitized samples and RAM means for storing data received from the converter means, characterised by a memory buffer of predetermined size in which the samples are stored continuously while the mean absolute value or absolute sum of a predetermined number of the samples is calculated so that, if the mean absolute value or absolute sum of the samples is below a predetermined threshold T1 and the buffer is filled with data samples, the oldest sample is discarded and a new sample is entered into the the buffer while, when the mean absolute value or absolute sum of the samples in the buffer is above threshold T1, the samples in the buffer and a predetermined number of subsequent samples, which together represent the transient waveform of the MES, are analysed using a computer algorithm to extract a predetermined set of transient waveform features from a predetermined number N of time segments, each M samples in length so that NxM is the total number of samples in the buffer, of the transient waveform, there being algorithmic means for comparing the transient waveform features against a group of feature sets stored in the RAM means whereby each stored feature set is indicative of a specific myoelectric transient pattern and is associated with a predetermined prosthetic limb function, the result of the comparison being a measure which is indicative of the similarity of the transient features to each of the stored feature sets.

2. A control system according to claim 1 which includes means for amplifying the differential voltage on the skin surface to obtain an analog myoelectric signal (MES).

3. A control system according to claim 2, in which the converter means comprise A/D converter means for receiving the analog MES and digitizing it at a predetermined sampling rate.

4. A control system according to any one of claims 1-3 which includes means for initiating the prosthetic function indicated by the result of the comparison.

5. A control system according to any preceding claim which includes means for controlling the prosthetic limb function in a manner proportional to the mean absolute value or absolute sum of the myoelectric signal calculated over a predetermined averaging window or predetermined number of samples.

6. A control system according to claim 5 which includes means for reducing the turn-on transient of the selected prosthetic function by processing the window sum or average, beginning at the point in time when the function has been selected, by a ramp or other smoothing function.

7. A control system according to claim 6 which includes means for comparing the mean absolute value or absolute sum to a predetermined threshold T2 so that, when the mean absolute value or absolute sum of the myoelectric signal is below threshold T2 for a predetermined time period, the prosthetic function is terminated.

8. A control system according to claim 7 which includes means for initiating the transient analysis after the function termination.

9. A control system according to any preceding claim which includes means for modifying the stored feature sets which are indicative of specific MES transient waveforms as well as means for indicating which prosthetic function is to be associated with each stored feature set.

## Patentansprüche

1. Myoelektrisches Steuersystem für ein künstliches Körperglied, umfassend Mittel, die angepasst sind, in Abhängigkeit von der dynamischen Information zu arbeiten, die sich in einem myoelektrischen Signal (EMG) befindet, das mit dem Beginn einer Muskelkontraktion verbunden ist, aus einem Konverter (A/D-Konverter; siehe Figur 1) zur Erzeugung von digitalisierten Proben und aus einem RAM-Speicher zur Speicherung von Daten des Konverters, **dadurch gekennzeichnet**, daß ein Pufferspeicher vorgegebener Größe vorgesehen ist, in dem die Proben kontinuierlich gespeichert werden, während der mittlere Absolutwert oder die mittlere absolute Summe einer vorbestimmten Zahl von Proben derart einer Berechnung unterworfen wird, daß die älteste Probe entfernt wird und eine neue Probe in den Pufferspeicher eingegeben wird, wenn der mittlere Absolutwert oder die mittlere absolute Summe der Probe unter einer vorbestimmten Schwelle T1 liegt und der Pufferspeicher mit Daten-Proben gefüllt ist und wenn der mittlere Absolutwert oder die mittlere absolute Summe der Proben in dem Pufferspeicher über der Schwelle T1 liegt, die Proben in dem Pufferspeicher und eine vorbestimmte Zahl nachfolgender Proben, die zusammen die transiente Wellenform des EMG bilden, unter Zuhilfenahme eines Computer-Algorithmus analysiert werden, um einen vorbestimmten Satz von transienten Wellenform-Merkmalen aus einer vorbestimmten Zahl N von Zeit Segmenten zu extrahieren, wobei jedes eine Länge von M-Proben hat, sodaß N x M die Gesamtzahl der Proben der transienten Wellenform in dem Pufferspeicher ist, daß ein Algorithmus-Teil dafür sorgt, daß die transienten Wellenform-Merkmale mit einer Gruppe von Merkmals-Sätzen verglichen werden, die in dem RAM gespeichert sind, wobei jeder gespeicherte Merkmals-Satz für ein spezifisches myoelektrisches Transienten-Muster charakteristisch ist und einer vorbestimmten Funktion eines künstlichen Körpergliedes zugeordnet ist, daß das Resultat des Vergleiches eine Größe ist, die kennzeichnend ist für die Ähnlichkeit der transienten Merkmale mit jedem der gespeicherten Merkmals-Sätze.

2. System nach Anspruch 1, **dadurch gekennzeichnet**, daß Mittel vorgesehen sind, die die Differentialspannung auf der Hautoberfläche verstärken, um ein analoges myoelektrisches Signal (EMG) zu erhalten.

3. System nach Anspruch 2, **dadurch gekennzeichnet**, daß als Konverter ein A/D-Konverter zum Empfang des analogen EMG und zur Digitalisierung desselben mit einer vorbestimmten Samplerate vorgesehen ist.

4. System nach einem der Ansprüche 1 bis 3, **dadurch gekennzeichnet**, daß Mittel vorgesehen sind, die diejenige prothetische Funktion initialisieren, die durch das Resultat des Vergleiches ausgewählt worden ist.

5. System nach einem der vorhergehenden Ansprüche, **dadurch gekennzeichnet**, daß Mittel vorgesehen sind, die die Funktion des künstlichen Körpergliedes proportional zu dem mittleren Absolutwert oder der mittleren absoluten Summe des myoelektrischen Signals, errechnet über ein vorbestimmtes Durchschnitts-Fenster oder eine vorbestimmte Zahl von Proben, steuern.

6. System nach Anspruch 5, **gekennzeichnet durch** Mittel, die die Eingangsschwankung der ausgewählten prothetischen Funktion reduzieren, indem die Fenster-Summe oder der Fenster-Durchschnittswert, beginnend mit dem Zeitpunkt, von dem an die Funktion ausgewählt wurde, durch eine Rampenfunktion oder eine andere Beruhigungsfunktion behandelt wird.

7. System nach Anspruch 6, **gekennzeichnet durch** Mittel, die den mittleren Absolutwert oder die mittlere absolute Summe mit einem vorbestimmten Schwellwert T2 vergleichen, so daß die prothetische Funktion beendet wird, wenn der mittlere Absolutwert oder die mittlere absolute Summe des myoelektrischen Signals für eine vorbestimmte Zeitdauer unter dem Schwellwert T2 liegt.

8. System nach Anspruch 7, **gekennzeichnet durch** Mittel zur Initialisierung der transienten Analyse nach der Funktionsbeendigung.

9. System nach einem der vorhergehenden Ansprüche, **gekennzeichnet durch** Mittel zur Veränderung der gespeicherten Merkmal-Sätze, die spezifischen EMG-Transient-Wellenformen zugeordnet sind, und durch Mittel, die anzeigen, welche prothetische Funktion einem gespeicherten Merkmals-Satz zugeordnet ist.

## Revendications

1. Un système de commande pour membre prothétique comprenant un moyen apte à fonctionner selon une information dynamique contenue dans un signal myoélectrique (MES) associé à l'apparition d'une contraction musculaire, le système comportant un moyen convertisseur (convertisseur A/N à la Fig. 1) pour produire des échantillons numérisés et un moyen de mémoire vive, ou RAM, pour mémoriser des données reçues du moyen convertisseur, caractérisé par une mémoire tampon de dimensions prédéterminées dans laquelle les échantillons sont mémorisés en continu, tandis que la valeur absolue moyenne ou la somme absolue d'un nombre prédéterminé des échantillons est calculée, d'une manière telle que l'échantillon le plus ancien est rejeté et un nouvel échantillon est entré dans le tampon si la valeur absolue moyenne ou la somme absolue des échantillons est inférieure à un seuil prédéterminé T1 et si le tampon est rempli d'échantillons de données, alors que les échantillons contenus dans le tampon et un nombre prédéterminé d'échantillons suivants, qui représentent ensemble la forme d'onde de transitoire du MES, sont analysés si la valeur absolue moyenne ou la somme absolue des échantillons est supérieure au seuil prédéterminé T1, en utilisant un algorithme d'ordinateur pour extraire d'un nombre prédéterminé N de segments temporels, d'une longueur unitaire de M échantillons telle que N x M soit le nombre total d'échantillons du tampon, de la forme d'onde de transitoire un ensemble prédéterminé de particularités de formes d'ondes de transitoires, un moyen algorithmique étant agencé pour comparer les particularités de formes d'ondes de transitoires à un groupe d'ensembles de particularités mémorisés dans le moyen de mémoire vive, grâce à quoi chaque ensemble mémorisé de particularités est indicatif d'une configuration spécifique de transitoires myoélectriques et est associé à une fonction prédéterminée du membre prothétique, le résultat de la comparaison étant une mesure qui est indicative de la similitude des particularités de transitoires avec chacun des ensembles mémorisés de particularités.

2. Un système de commande selon la revendication 1, qui inclut un moyen amplificateur de la tension différentielle sur la surface de la peau pour obtenir un signal myoélectrique analogique (MES).

3. Un système de commande selon la revendication 2, dans lequel le moyen convertisseur comprend un moyen convertisseur A/N pour recevoir le MES analogique et le numériser à une cadence d'échantillonnage prédéterminée.

4. Un système de commande selon l'une quelconque des revendications 1 à 3, qui inclut un moyen de lancement de la fonction prothétique indiquée par le résultat de la comparaison.

5. Un système de commande selon une revendication précédente quelconque, qui inclut un moyen de commande de la fonction du membre prothétique d'une manière proportionnelle à la valeur absolue moyenne ou à la somme absolue du signal myoélectrique, calculée sur une fenêtre prédéterminée de calcul de moyenne ou sur un nombre prédéterminé d'échantillons.

6. Un système de commande selon la revendication 5, qui inclut un moyen de réduction de la transitoire de mise en marche de la fonction prothétique sélectionnée, en traitant par une rampe ou une autre fonction de lissage la somme ou la moyenne de la fenêtre en débutant à l'instant où la fonction a été sélectionnée.

7. Un système de commande selon la revendication 6, qui inclut un moyen de comparaison de la valeur absolue moyenne ou de la somme absolue à un seuil prédéterminé T2 de façon à terminer la fonction prothétique lorsque la valeur absolue moyenne ou la somme absolue est inférieure au seuil T2.

8. Un système de commande selon la revendication 7, qui inclut un moyen de lancement de l'analyse des transitoires après la terminaison de la fonction.

9. Un système de commande selon une revendication précédente quelconque, qui inclut un moyen de modification des ensembles mémorisés de particularités qui sont indicatifs de formes d'ondes de transitoires spécifiques de MES, ainsi qu'un moyen d'indication de la fonction prothétique qui doit être associée à chaque ensemble mémorisé de particularités.
